# EUROPEAN PATENT SPECIFICATION

(11) **EP 2 175 276 B1**
(45) Date of publication and mention of the grant of the patent: **08.08.2012**
(21) Application number: 09175570.2
(22) Date of filing: 31.10.2005
(51) Int. Cl.: G01N 33/66, C07F 5/02

(54) **Raman-detection based analysis for glucose products using pyridinylboronic acid**
Raman-Nachweis Analyse für Glucoseprodukte unter Verwendung von Pyridinylborsäure
Analyse de produits du glucose à l'aide de l'acide pyridinylboronique par détection Raman

(30) Priority: 29.10.2004 US 623472 P
(43) Date of publication of application: 14.04.2010
(62) Divisional of application: 05819895.3
(73) Proprietor: The University of Akron, Akron, OH 44325 (US)
(72) Inventor: Hu, Jun, Fairlawn, OH 44333 (US)
(74) Representative: von Kreisler Selting Werner

(56) References cited:
- EL KHOURY JOULIANA M ET AL: "Indirect detection of ggucose in an aqueous solution using surface-enhanced Raman scattering." ABSTRACTS OF PAPERS AMERICAN CHEMICAL SOCIETY, vol. 228, no. Part 1, August 2004 (2004-08), page U472, XP008100019 & MEETING OF THE DIVISION OF CHEMICAL TOXICOLOGY OF THE AMERICAN-CHEMICAL-SOCIETY HELD AT THE 228TH N; PHILADELPHIA, PA, USA; AUGUST 22 -26, 2004 ISSN: 0065-7727
- KANAYAMA N ET AL: "Interfacial recognition of sugars by boronic acid-carrying-self-assembled monolayer" LANGMUIR, AMERICAN CHEMICAL SOCIETY, WASHINGTON, DC, US, vol. 16, no. 2, 25 January 2000 (2000-01-25), pages 577-583, XP002997117 ISSN: 0743-7463
- CLARK HALEY R ET AL: "Modeling the response time of an in vivo glucose affinity sensor" BIOTECHNOLOGY PROGRESS, vol. 15, no. 2, March 1999 (1999-03), pages 259-266, XP002569362 ISSN: 8756-7938

## Description

### BACKGROUND OF THE INVENTION

This invention relates to detection of glucose and related sugar products using pyridinylboronic acid. The analysis can be via a colormetric method, a spectroscopic method or a spectrometric method, here raman spectroscopy is used.

Molecular recognition of sugars by arylboronates in aqueous solutions involves reversible covalent bonding. Design of new boronic acid based sugar sensing scaffolds remained an active field of research because the molecular recognition process is not fully understood despite of the extensive investigations. While many recent studies have been focused on bisboronic acids, the search for the highest sugar binding monoboronic acid motif continued for understanding the structures and energetics of the bindingprocesses and for providing more effective building blocks in designing more sophisticated molecular sensors. The main reason for developing bisboronic acid sugar sensors is to differentiate glucose and fructose because most of the monoboronic acids bind to glucose about 50 times better than to fructose under typical sensing conditions.

Real-time chemical monitoring and imaging techniques are of fundamental interests in scientific inquiries of biological functions, disease early diagnoses, critical care patient monitoring, and long-term self-management of chronicle diseases. Raman spectroscopy is one of the most promising techniques for *in vivo* optical bio-sensing and biochemical imaging due to its high specificity and compatibility to biological samples. Recent advances in instrumentations and sampling techniques have led to dramatic improvement in Raman spectroscopy sensitivity, rendered it comparable to that of fluorescence spectroscopy in favorable cases. See e.g El khoury J.M. et al Abstracts of papers american chemical society, vol.228, part1, page U472, 22 august 2004.

Real-time *in vivo* monitoring of vital metabolites such as glucose in critical-care patients has been a long-standing and elusive goal in biosensor design and fabrication.

The use of chemical stains to improve contrast of the pathological markers in Human tissues for optical microscopic imaging is a well-established procedure in biomedical research and clinical analysis. In fluorescence imaging, fluorescence probes are used routinely to create distinctive colorful emission contrast. Molecular detection or imaging is achievable in fluorescence sensing or microscopy when the emission probes are conjugated to targeting moieties such as antibodies. In contrast, the use of chemical enhancement agents in Raman sensing and imaging in biomedical applications is underdeveloped.

Raman spectroscopy is considered one of the most powerful *in vivo* methods to obtain molecular structure information of bio-systems when coupled with optical microscopy and fiber optics. Raman spectroscopy provides much chemical and biochemical information of the specimens as it monitors the molecular structures and their changes from molecular vibrational "finger prints". Its intrinsic selectivity is much greater than that of other optical methods such as absorption and fluorescence detections. However the low Raman sensitivity of most biomolecules in soft tissues limits its applications. Biosensors and related microanalytical systems represent a potentially explosive market for analytical instruments that are portable, smart, and suitable for mass production. These characteristics are desirable or even critical for many healthcare and environmental related applications such as monitoring glucose level in diabetic patients, detecting nerve gas and biological agents in battlefields, monitoring air and water quality, food safety, disease diagnosis, and drug discovery. From a long-term perspective, biosensors will play a key role in disease prevention, early diagnosis and intervention, and long-term self-management of chronic illnesses.

A sensor-based bioanalytical system often constitutes a transducer such as an electrode or optode, electronics and/or optics, and a microcomputer. Integration of all these components into a clinical device or a consumer product is a challenging engineering task itself, yet the key to the success of such a device is often the transducer, *i.e.,* the chemical or biological sensing interface that responds to the concentration of a chosen analyte. Many ingenious designs have been developed for coupling chemical and biological events at sensing interfaces into detectable electronic or optical signals. Most of these designs rely on or mimic nature's molecular recognition schemes such as enzymatic reactions and receptor-ligand bindings. Despite enormous research efforts, the number of sensors for real-time monitoring of complex mixtures is still quite small, and biosensors suitable for *in vivo* applications are quite rare.

There are many formidable challenges in fabricating the sensing interface for an implantable biosensor: First, a practically useful sensor should have a quantifiable response to the concentration of a specific analyte in the presence of interferences from the sample matrix, which is usually a very complex mixture. This requires the sensing element to be highly specific. Second, the response should also cover the analyte concentration range that is relevant to the specific sensing application. Therefore, the sensing interface should have a sufficiently high sensitivity and a wide dynamic range. Third, the response should also be rapid and reversible such that the readout represents the "real-time" analyte concentration. Fourth, the sensor interface should have a sufficiently long lifetime (several days to months), and during this period it should be stable and easy to calibrate. In addition, the sensing interface should be biocompatible for "long-term" real-time applications to avoid complications from materials-induced bioresponses such as nonspecific protein binding, inflammation, and wound healing. Furthermore, it is desirable that the sensing interface is suitable for miniaturization, sterilization and mass production for eventual success in clinic or marketplace.

Glucose sensors have been the subject of intensive studies mainly due to their importance for diagnosis and treatment of diabetes. Recently the International Diabetes Federation reported that there are over 177 million diabetics worldwide with a potential of dramatic increases in developing countries.

Hand-held devices based on mediated amperometric sensors are now in clinical use for diabetes patients. These sensors are built on the principles of oxidation of glucose catalyzed by a glucose oxidoreductase enzyme. The enzyme-modified electrode detects the electrons generated in the enzymatic reaction through an electron coupler such as ferrocene that is also bound to the electrode surface. These devices provide convenient one-shot measurements of glucose in a pinpricked blood sample. The successful development and commercialization of the electrochemical glucose sensors have provided diabetic patients essential means for self-management of such a chronic disease. Yet long-term implantable glucose sensors suitable for minimally invasive or noninvasive repeated real-time detections have not been realized despite a tremendous amount of research. The main difficulties include short lifetimes of enzymes, complications from enzyme cofactors and the bio-incompatibility of the sensing interfaces. The high cost for fabrication and complexity in calibration render the mass production of these sensors difficult. In addition, biosensors made of enzymes and other biomaterials are usually not compatible with the common sterilization methods required for *in vivo* applications.

"Gluco Watch" based on iontophoretic extraction of body fluid through skin has been developed for minimum invasive monitoring of blood glucose. This approach has a significant time delay between blood glucose concentration and readout and suffers from several calibration disadvantages. Current strategies under investigations for continuous noninvasive glucose monitoring also include near-infared (NIR) spectroscopy, and colorimetric contact lens type of sensors, and implantable sensors.

From a spectroscopic point of view, glucose sensing and sugar analysis in general in biological fluids represents a "Holy Grail" in bioanalytical science. Sugar molecules usually display very low optical densities and spectroscopic signatures in aqueous solutions. Direct spectroscopic measurements are complicated by peak broadening due to the strong hydrogen bonds and conformation changes in aqueous solutions. As a promising alternative to enzymatic reaction-based sensing methods, affinity sensing that utilizes synthetic "receptors" as spectroscopic transducer units is considered the most promising "implantable" approach. As in receptor-ligand or antibody-antigen interactions, the molecular recognition processes in this type of sensing mechanisms involve no chemical reactions, and the difficulties in quantifying cofactor effects on reaction rates are therefore eliminated. Affinity binding is one of the most widely applicable mechanisms in sensor designs that allow for relatively easy coupling with optical and electronic detecting methods.

Reversible covalent complexation between phenylbronic acid and diols has been studied extensively for developing affinity base glucose sensors. Other commonly used molecular recognition techniques such as hydrogen-bonding interactions are usually ineffective in these conditions. Glucose, boronic acid, boronate, boronic esters, and other acidic-basic species form complex equilibriums in an aqueous solution (Scheme 1). Chelating of diols to the boronic acid increases the acidity at the boron and favors the formation of the boronate-diol complex. It is generally accepted that the boronate-diol complex is the dominant species in a boronic acid-diol solution if the pH of the solution is above the pKa ofthe boronic acid. In the favorable cases, the intrinsic sensitivity of proper spaced bisboronate scaffold-based glucose sensors already rivals that of enzyme-based sensing methods.

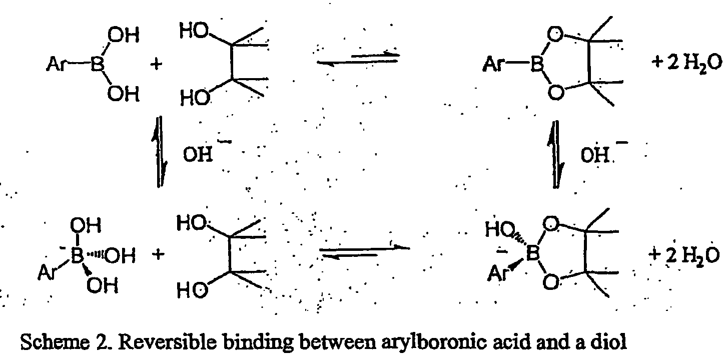

The binding events between boronic acid and glucose was initially been incorporated into fluorescence spectroscopy based optical glucose sensors. For example, Shinkai et al (Shinkai, Seiji and James, Tony D.; "Molecular & Supramolecular Photochemistry", 2001, 7, 429-456) used electron transfer quenching of fluorescing excited state as a modulator to readout the glucose binding (Scheme 3). James et al (James, T. D.; Sandanayake Samankumara, K.R.A.; and Shinkai, S.; "Angewadrte Chemie, International Edition; 1996, 35, 1911-1922; and Supramolecular Chemistry, 1995, 6, 141-157) developed a fluorescence resonance energy transfer (FRET) sensor for glucose base on a bisboronic acid glucose binding scaffold. Colorimetic detections using competitive titrations with a diol dye, and boronic acid modified photonic crystals are also feasible alternative optical readout methods.

Nonspecific adsorption of biomolecules at the molecular recognition sites is one of the severest problems in developing affinity biosensors. The reaction sites of enzyme-based sensing interfaces are almost always well protected by the bulk of the protein matrix so that deactivations due to nonspecific binding are rare. To achieve access-control and biocompatibility of a sensing interface, encapsulation of the sensing interface within a protective polymer shell represents a viable biomimetic approach to this problem.

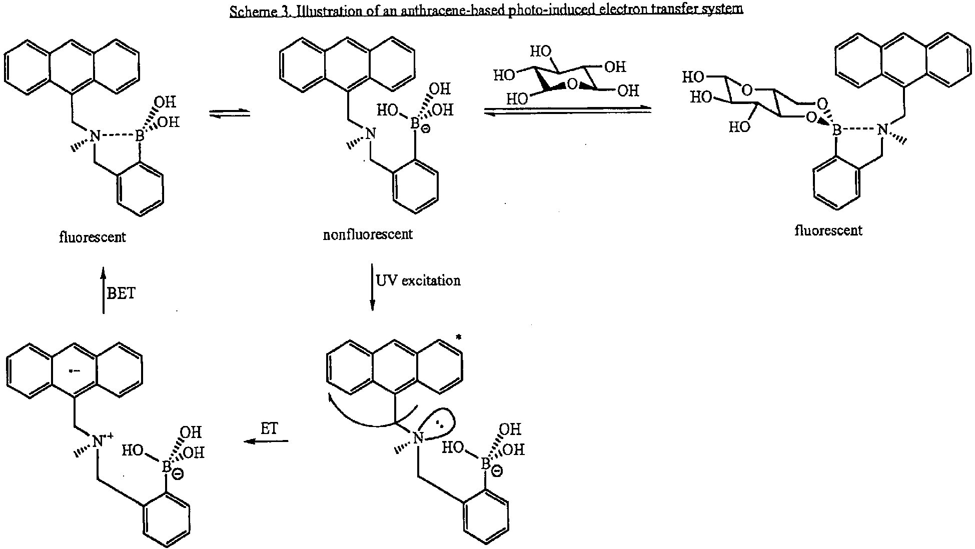

Over the past decade, much research has been devoted to electron transfer fluorescence quenching sensors for glucose based on benzylaminoboronic acid as shown in Scheme 3. This method suffers from two chemical structural difficulties : (1) The energy of the emitting fluorophore has to match that of the nonbonding electrons of the amino group for electron transfer fluorescence quenching. This requires that the excitation light to be at UV where biological molecules also absorb and fluoresce. (2) Benzylboronic acids usually bind to glucose at above pH 8. At physiological pH, protonation at the amino group occurs to compete with boron coordination.

### SUMMARY OF THE INVENTION

Our invention as defined by the claims is the result of the discovery that the dynamic covalent supramolecular bonding, between the pyridinylboronic acid and diols or triols in aqueous solutions, allows convenient in situ derivatization of the sugar analytes; and the zwitterionic nature of arylboronic acid allows intense ion signals of both the corresponding cations and anions to be observed by a simple switching of the ESI-MS polarity. In addition, the unique mode of complexation of arylboronic acid with sugars, i.e. its preference for triols in the neutral aqueous solutions, allows for the differentiation of glucose from fructose in direct ESI-MS and ESI-MS-MS (ESI-MS2) employing an ion-trap mass spectrometer.

In general the present disclosure provides method of analyzing a target analyte using laser excitation spectroscopy, wherein the laser excitation spectroscopy is enhanced using an pydinylboronic acid.

The present disclosure also includes contrast enhancement agents that are specifically designed for molecular recognition-based Raman spectrographic biosensing and molecular/chemical imaging.

The present disclosure can be employed in performing colormetric analysis using pyridinylboronic acid by colormetric titration of glucose with the pyridinylboronic acid and a visible catechol dye, such as for example, pyrocatechol violet.

### BRIEF DESCRIPTION OF THE DRAWINGS

Figure 1 is a calibrations curve for visible colorimetric competitive titrations of monosaccharides in aqueous phosphate buffer solutions at pH 7.4;
Figure 2. Surface Enhance Raman Spectra on 60 nm gold: (a) 3-pyridinylboronic acid with glucose, and (b) 3-pyridinylboronic alone sampled from aqueous solutions;
Figure 3 is a colorimetric quantification of fructose; and

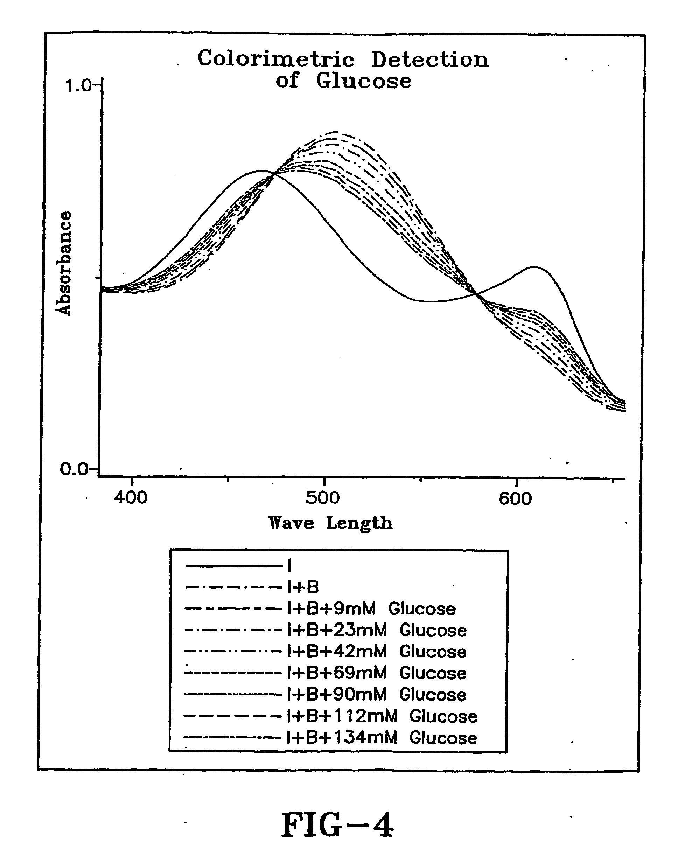

Figure 4 is a colorimetric quantification of glucose.

### DETAILED DESCRIPTION OF ILLUSTRATIVE EMBODIMENTS

New optical glucose molecular sensors can be designed and synthesized for long-term use as a microscopic "tattoo dye" for non-invasive Raman readout of interstitial glucose concentrations. The new glucose sensing elements should also be applicable to other bioanalytical applications involving molecular recognition of diols.

Several arylboronic acid derivatives are identified. They (1) selectively bind to biomolecules with 1 ,2- and 1 ,3-diols; (2) show specific changes in Raman spectra before and after complexations; (3) display high Raman sensitivities alone or indirectly with additional chromophors; and (4) self-assemble on gold and silver nanoparticle surfaces thus potentially useful for surface enhanced Raman analysis. These facilitate using such molecules as chemically selective Raman enhancement agents for detection and quantification of glucose in water under physiological conditions. The proposed supramolecular design, synthesis, and Raman applications of molecular sensors are fundamentally innovative.

3-pyridinylboronic acid is a zwitterionic arylboronic acid having a remarkable sugar binding motif which allows for its use as a colorimetric quantification method for glucose at neutral pH. The unusually high sugar affinity of arylboronic acid is due to its preference for triols rather than diols, a manifestation of an undocumented stereoelectronic effect in this arylboronic acid. The preference for tridentate complexation of arylboronic acid is advantageous in sugar sensing sensitivity and selectivity from both thermodynamics and stereochemistry point of views.

Arylboronic acid based molecular sensor for glucose can be used with a microscopic, none-enzymatic, implantable optical sensor based on polymer encapsulated pyridinylbronic acid and derivatives. The polymer capsule is designed to be biocompatible or biodegradable hydrogels, and noninvasive colorimetric and Raman spectroscopic read out of the reversible binding events of the sensors will be demonstrated. This allows the use of chemical enhancement agents for *in vivo* sensing and molecular imaging using Raman spectroscopy/spectromicroscopy.

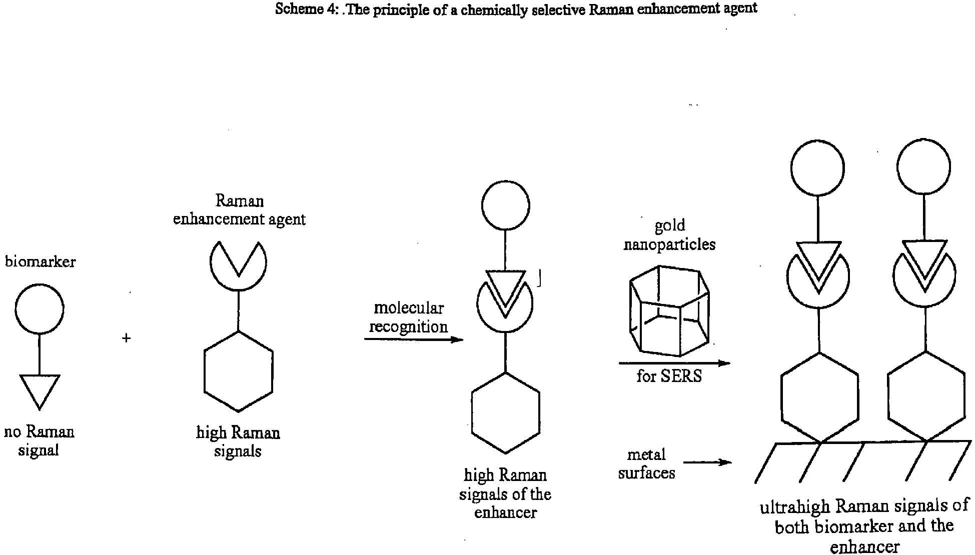

The principle of the proposed chemically selective Raman enhancement agent is shown in Scheme 4. A Raman inactive or low sensitive metabolite or biomarker of interest may be detected by using a molecular sensor that (1) selectively binds to the biomarker, and (2) displays high Raman signals. Furthermore, Raman enhancement agents can be designed to allow resonance Raman and/or surface enhanced Raman (SERS) to achieve ultrahigh sensitive detections. The present invention employs boronic acid based affinity glucose sensors.

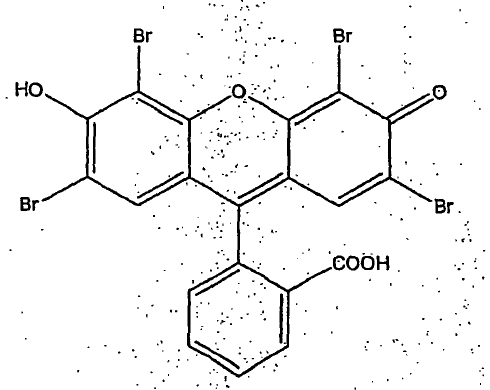

Formula 5 is an example of encapsulate a fluorescence small molecule (Eosin Y) in the core-shell polymer: red fluorescence emission from bright field optical microscopy imaging with green light illumination

The polymeric particles loaded with an acidic fluorescent dye showed a clear intensity gradient ofthe fluorescence. The maximum intensity was in the center ofthe particle which gradually fades out along the radius. This indicates that the core of the polymer particle is loaded with the dye molecules. Gold nanoparticles suitable for SERS studies may also be deposited using standard reduction method of HAuCl₄ loaded in the polymer core shell particles.

Hollow spherical structures or encapsules can be integrated into supramolecular biosensors and biocompatible materials via nanoparticle-based microfabrications to encapsulate sensing materials that can be applied as "tattoo dyes" for noninvasive readout by optic detection from outside of the body.

The biocompatibility of the shell materials must be considered if our ultimate goal is to develop a sensing material for long-term *in vivo* applications. Implantation of a sensor in the body of a patient may induce adverse reactions at the site where the sensing interface contacts with living tissues. It is often the case that a "biosensor" may work perfectly in detecting an analyte in a simple solution and fail completely when it is used in direct contact with body fluids. Biofouling by nonspecific and irreversible adsorption of proteins, inflammation, fibrous encapsulation, and sensor interface biodegradation are among the common causes for this failure in long-term use of an implantable biosensor. In biosensor design, we must consider biocompatibility, i.e., we must seek to develop a sensor interface that will cause minimum reactions in the body yet remain operational in the biological environment. The achievable small size of our optical sensor should be advantageous for inflicting minimal wounds during implantations. Further improvement of the performance of the materials may be achieved by masking the nanocapsules biocompatible.

Several coatings have been developed to improve the biocompatibility of macroscopic biosensors. Hydrogel overlay, phospholipid-based biominicry, co-deposition of bioactive and biocompatible components, and covalent attachment of biocompatible materials are the common strategies for improving the biocompatibility of an implant. Hydrogel coatings have been shown to reduce biofouling of implants. Water-swollen polymers allow glucose to diffuse freely to the sensor while hindering protein adsorption due to the energetically unfavorable displacement of water by the encroaching protein and the subsequent polymer compression. The most widely used hydrogels are cross-linked polymers of either poly(hydroxyethyl methacrylate) (PHEMA) or poly(ethylene glycol) (PEG).

An alternative strategy is to coat the biosensor with polymers that mimic cell membranes such as phospholipid-like biomaterials. If the sensor appears as a natural cell, it may elicit less of an immune system response. However, such materials may not be optimal for glucose sensing because the solubility of sugar in the typical hydrophobic lipid-like materials is rather poor and the mass transportation of glucose in such materials is expected to be very slow.

Encapsulation of the molecular recognition sites prevents the sensing elements from direct contacting with macromolecules that bind to the sensing interface nonspecifically. The protective shells can also improve biocompatibility of the sensing interface that is essential for long-term *in vivo* use of implantable biosensors.

Our polymer encapsulation approach can also be coupled with the SERS detection. The nano gold cluster can be generated within the polymer capsule. The enhanced optical field near the gold nanoparticle surface will "light up" the molecular recognition interface providing selective SERS for the binding sites.

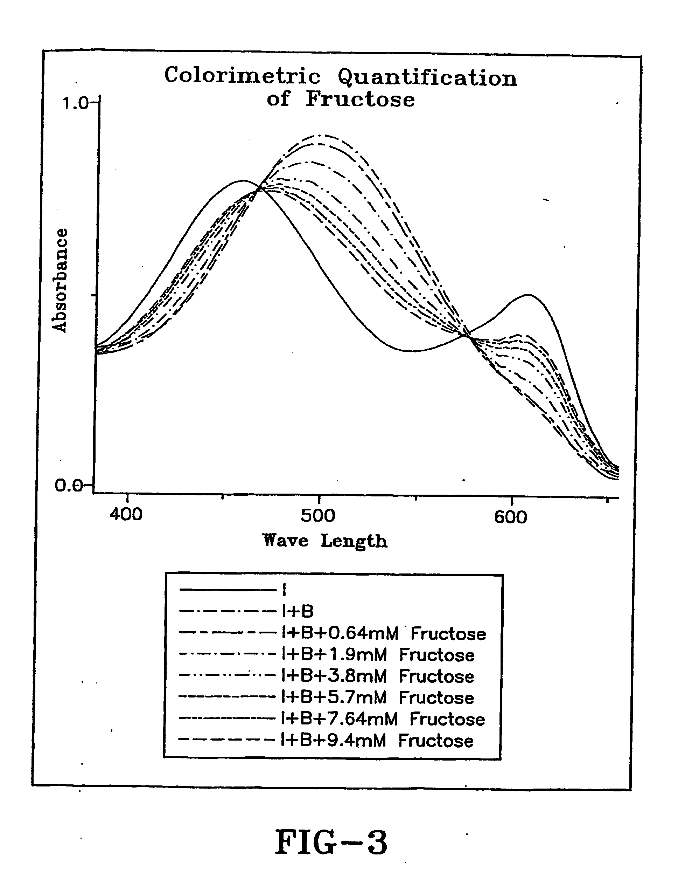

To demonstrate a colormetric analysis, a solution of 3-pyridinylboronic acid (1, 6.60 • 10⁴ M) and pyrocatechol violet (3,7.54 · 10⁵ M) in phosphate bufer (3.0 ml, 0.01 M, pH 7.40) in a quartz cuvette (10.0 mm) was titrated using an aqueous solution of glucose (2.81 M in 5.00 1L portions using a Fisher Science microdispenser). The titration was monitored with an OceanOptics_S2000 spectrometer at 25 °C. The pH of the solution was checked to be steady by a pH meter (Oakton 510). The apparent 60 binding constant was determined by using the competitive essay binding algorithm and the method reported by Tobey and Anslyn. The addition of glucose solution led to absorbance peak shifts and intensity changes of the solution that can be easily quantied with a low profle fiber-optic spectrometer. The dye displayed two absorption peaks in the buffer solution due to the acid-base equilibrium. When 3-pyridinylboronic acid was added, a complex was formed as the predominant boronic ester with a kmax of 503 nm, 70 redshifted from the conjugated acid form of the dye (kmax = 463 nm). It can be estimated from the data that glucose of a few millimolar in neutral aqueous solutions can be reliably quantifed using this assay. The 50 nm redshift of the absorbance peak and the 20% increase in molar absorbance of the dye used in titration were attributed to an extension of the π-conjugation of the dye to the arylboronic acid moiety in the boronic ester. 11B NMR of the complex dis-80 played a single peak at 18.8 ppm, indicating that the boron in the ester is predominantly trivalent (5, sp2). This signifcant increase in the absorbance of the dye improves the sensitivity (S/N ratio) of colorimetric detection. The high binding afnity was confirmed from NMR studies at 25°C. For example, 1H NMR spectrum of a mixture of 1 and glucose (15 mmol 1 and 10 mmol glucose, 0.70 ml D2O) showed two broadened anomeric hydrogen peaks at 6.1 and 5.9 ppm, which were assigned 90 to (a-D-glucofuranose)Æ1 (6) and (a-D-glucofuranose)Æ12 (7 and 8), respectively (Fig. 3 and Scheme 2). When 4 equiv of 1 were used, we observed that the peak at 6.1 ppm disappeared and the 5.9 ppm resonance became dominant. The peaks at 5.3 and 4.7 ppm are assigned to the anomeric hydrogen signals of uncomplexed glucose. From the integrations (Fig. 3), the concentrations of free glucose (1), the 1:1 complex (6), and the 1:2 complex (7and 8) can be determined to be 4.2,7.1, and 3.8 mM, respectively. With an error in NMR integration estimated to be less than 5%, it can be calculated from the integrations of the anomeric proton peaks that the apparent binding constant for the formation of 6 is about 130, consistent with the colorimetric titration described above. In comparison, the 1H NMR spectrum of a mixture of N-methylpyridinium-3-boronic acid (28 mM) and glucose (7 mM) showed the complexed furanose anomeric hydrogen peak at 5.8 ppm. Less than 5% of the glucose in the sample is estimated to be complexed from the NMR integration in pH 7.4 phosphate buffer 110 in D₂O at 20 °C.

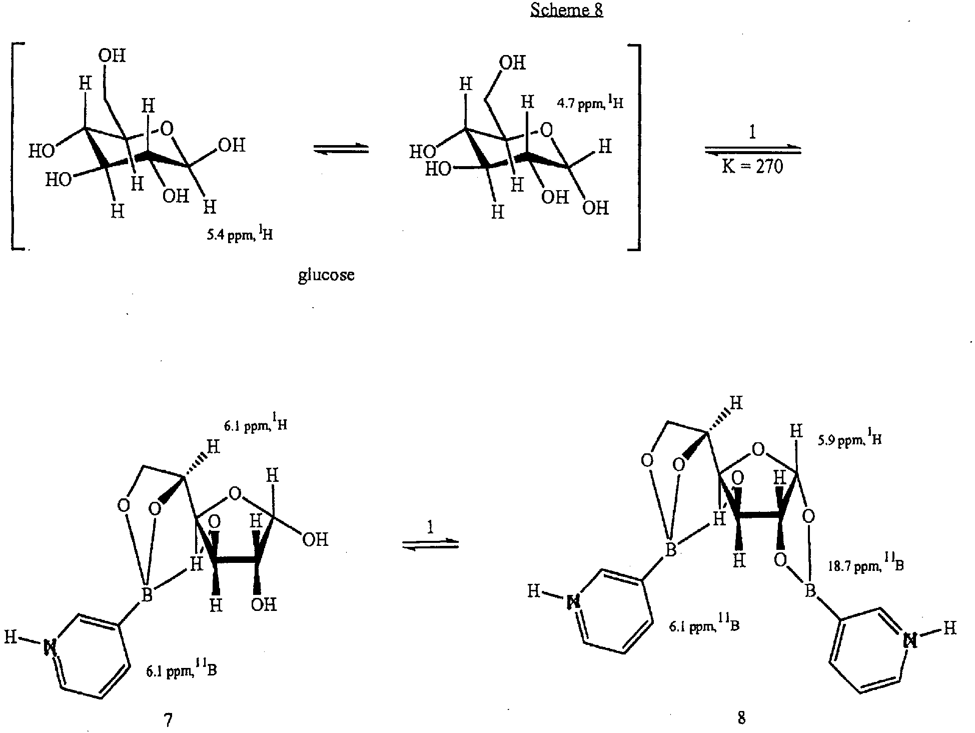

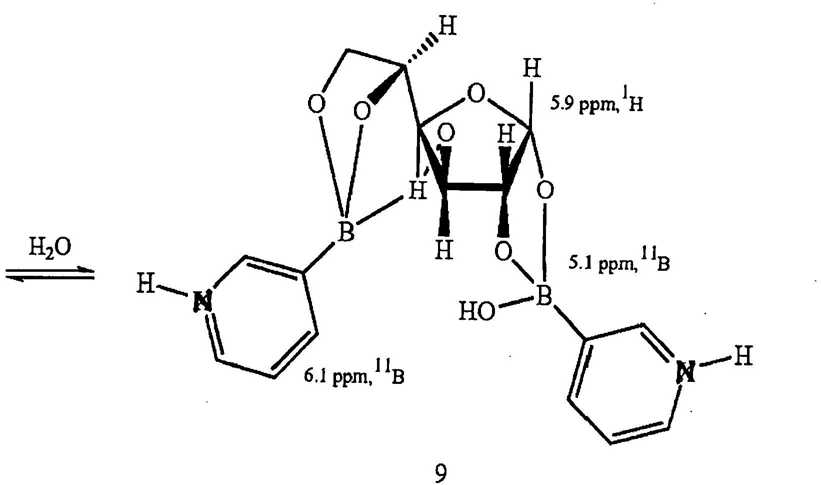

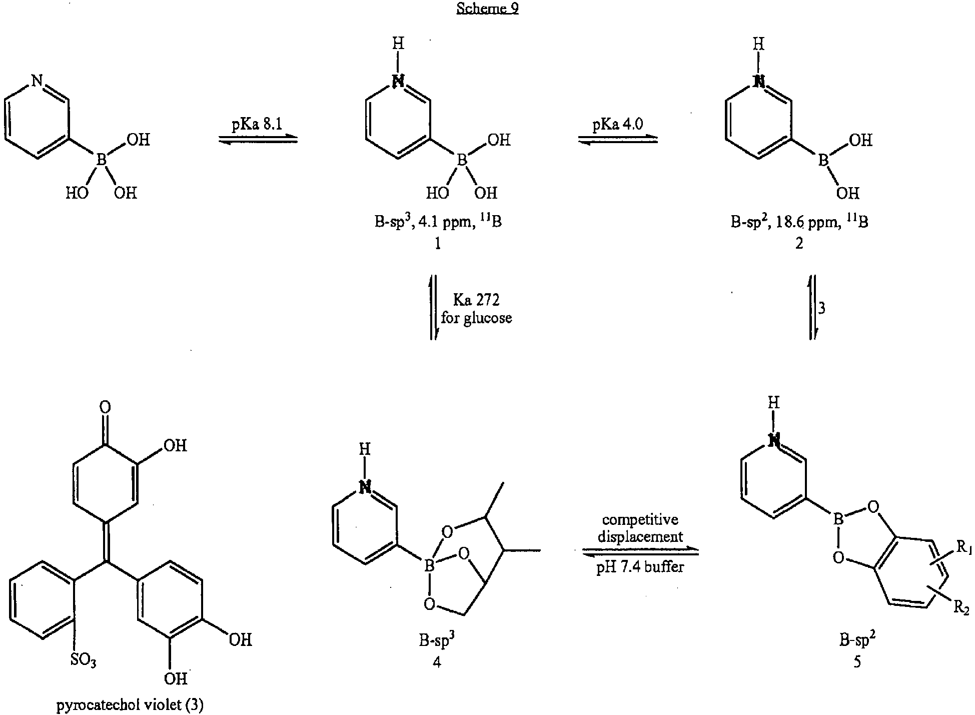

As seen in scheme 9 the bonding is a triol bonding. 3 -pyridinylboronic acid, substituted boronic acids, and derivatives can be used for engineering an implantable Raman glucose sensor and for use as Raman enhancement agents for molecular biomedical imaging in general. This allows for the well-known molecular recognition interactions between aryboronic acid and diol.

The required high Lewis basicity of the amino group for boron coordination contradicts the required low Bronsted basicity of the amino group for aqueous physiologic pH operations. In addition, the energetics of the electron transfer quenching which intrinsically determined by the boron p orbital energy can not be further tuned to longer wavelengths for sensing and imaging at biological conditions (*in vivo).*

A new sensing element for glucose was thus designed, synthesized, and characterized based on pyridinylboronic acid (Scheme 4). The dilemma between Lewis basicity and Bronsted basicity for the prevailing arylbronates used for sugar sensing is resolved by incorporating the electron withdrawing nitrogen in the aromatic ring. Protonation of the pyridine nitrogen at low pH further increases the inductive electron withdrawing effect of the nitrogen over the aromatic ring and hence increases the Lewis acidity of the boronic acid comparably. This approach in sugar sensor design is fundamentally innovative and should be applicable to various kinds of affinity based sensing and imaging techniques for sugars. In this proposal the optical biocompatibility of the glucose sensor is addressed by using visible colorimetric or Raman detection. The excitation wavelengths for Raman and surface enhance Raman can be tuned to avoid fluorescence background of biomolecules and allow sufficient optical throughput through biological samples. The materials biocompatibility ofthe glucose sensor will be addressed using polymer gel encapsulation of the boronic acid and indicator.

The selectivity of detection of glucose over other sugars in blood in principle can be achieved by the intrinsic spectroscopic signatures in the Raman spectroscopy. However, the results indicated that the Raman active modes of the glucose part of the complex are particularly weak and broadened. Water soluble, low florescent bisboronic acid glucose binding motifs, such as for example, bisboronic acid with varied spacers between the 3-pyridinylboronic acid and as shown in Scheme 6 are preferred. These compounds are selected based on reported selectivity for glucose binding bisboronic acids and the synthesis are straight forward as show in the Scheme 6.

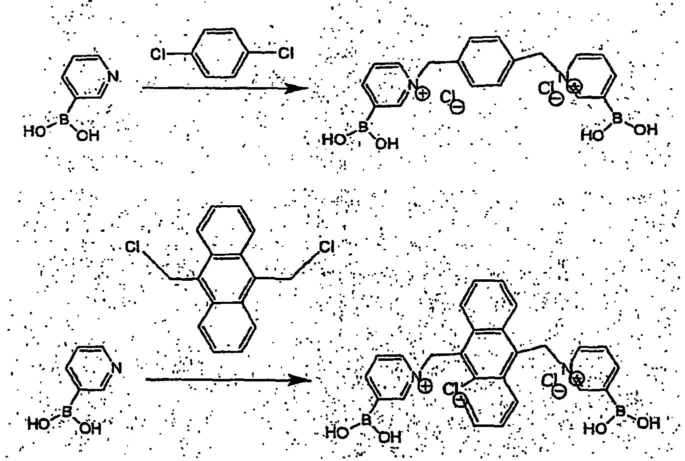

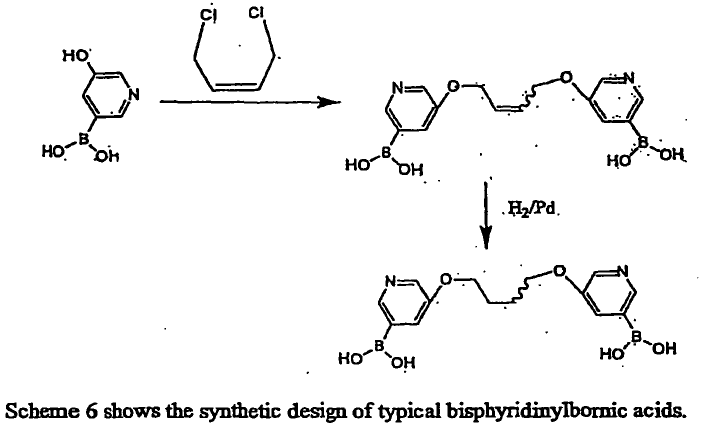

The development of polymer core shell encapsulated optical glucose sensors having use in visible colorimetric and Raman spectroscopic quantifications of glucose in physiological conditions is shown in Scheme 7, including the preparation of polymer core shell encapsulated glucose sensors with gold nanoparticles within the core and hydrogel shells for surface enhanced Raman sensing of glucose.

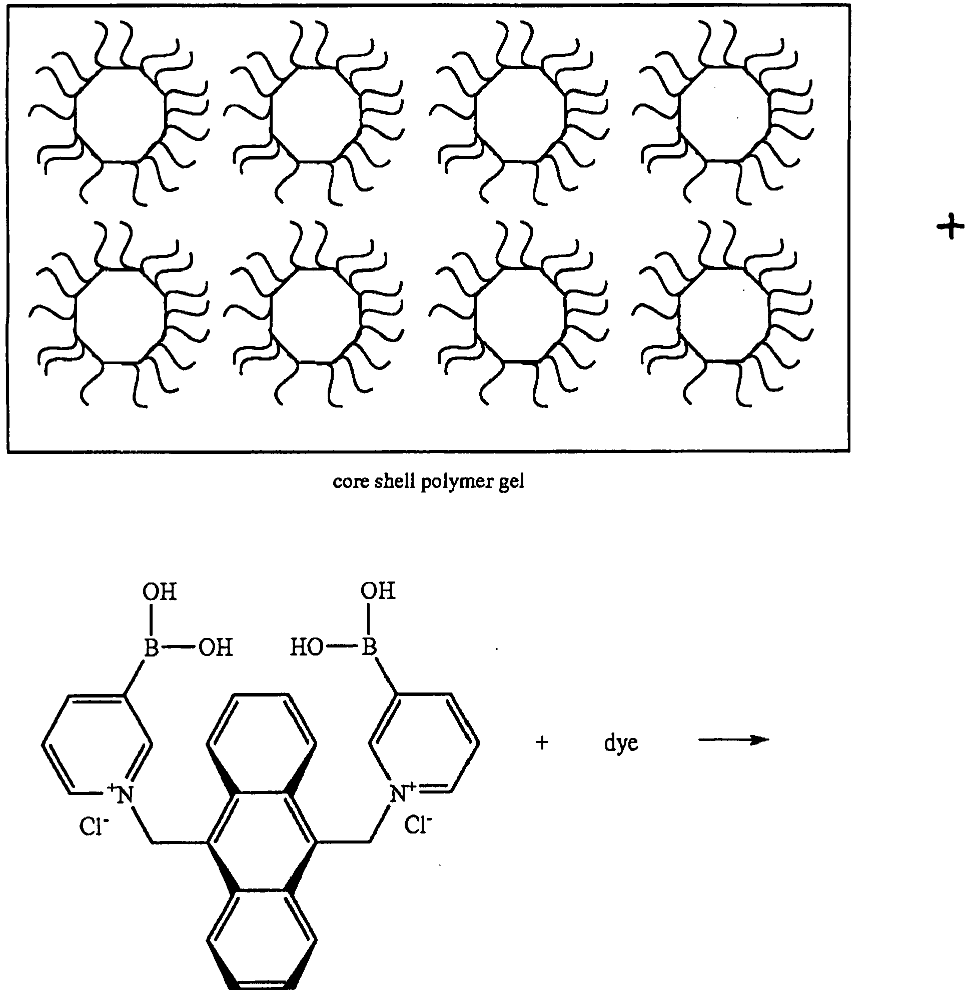

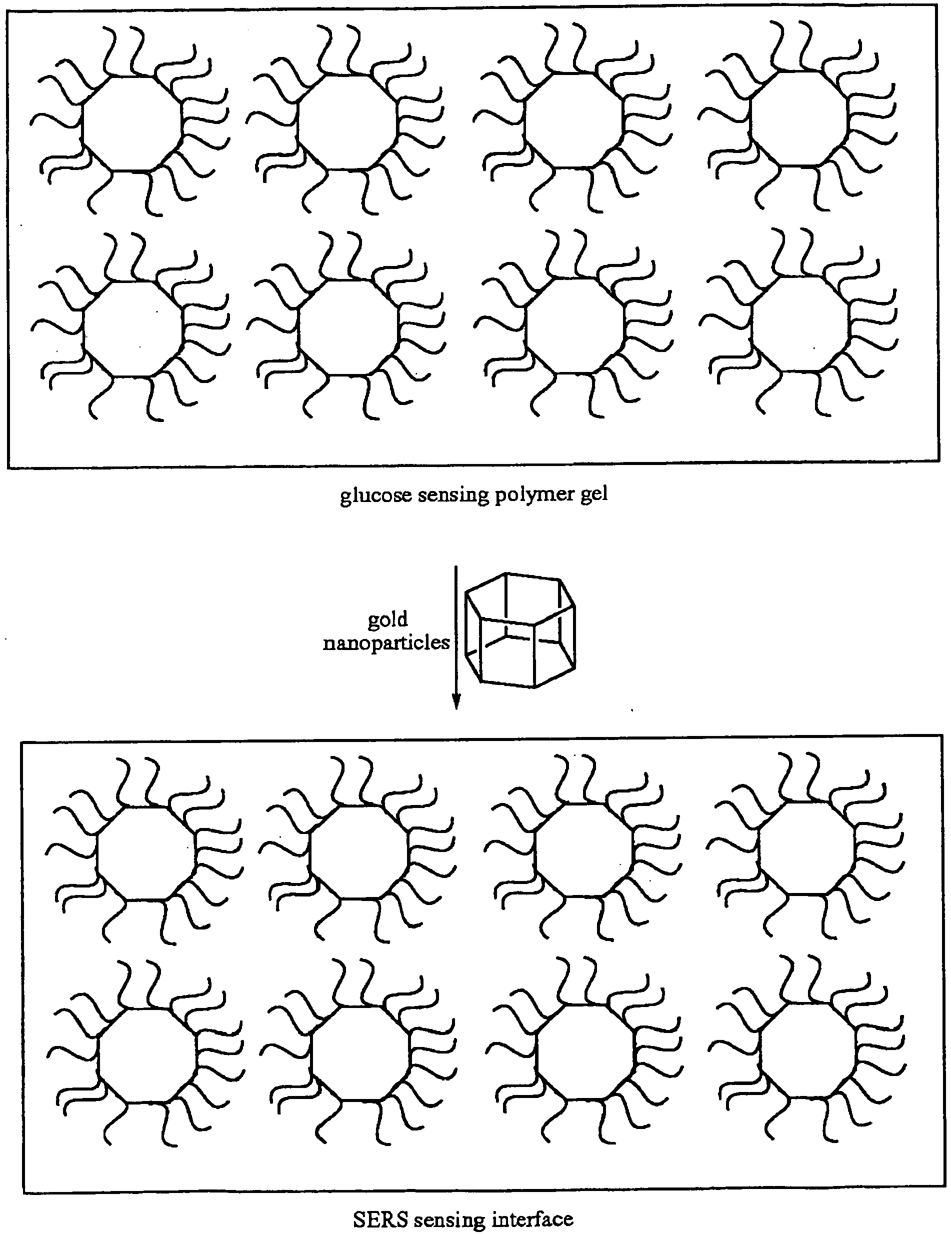

Figure 7 is a design of the Bisboronic Acid and Raman Glucose Sensing Polymer Hydrogel

This results in a polymer material that is suitable for evaluations as an implant or a contact lens to achieve noninvasive, real-time detections of glucose in *vivo.*

In order to demonstrate the practice of the present invention, the following examples have been prepared and tested. The examples should not, however, be viewed as limiting the scope of the invention. The claims will serve to define the invention.

### EXAMPLES

### Example 1

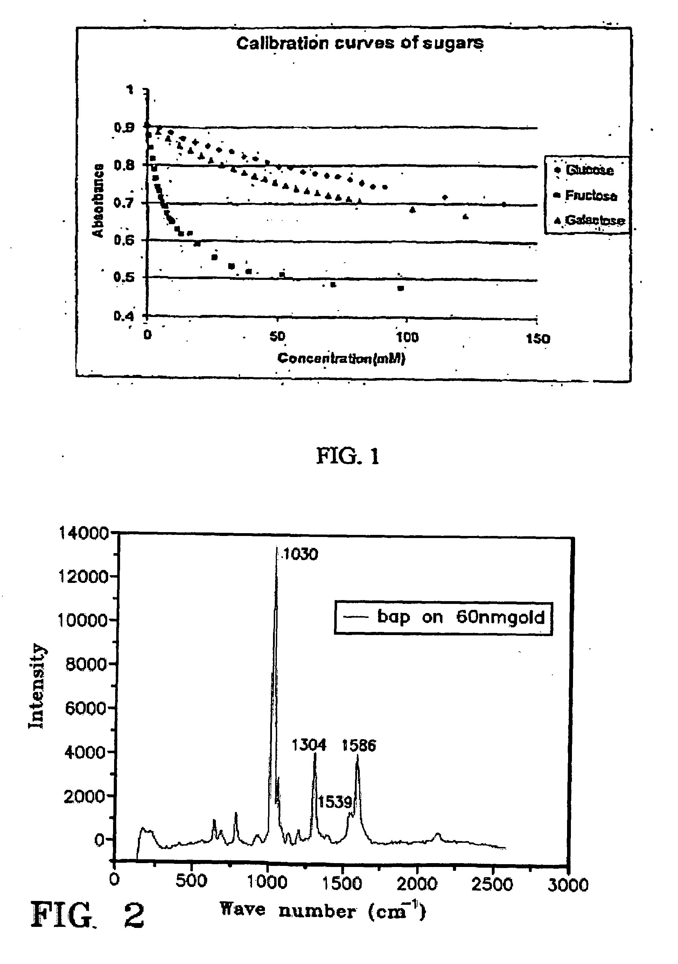

The Raman and surface enhanced Raman spectra of several pyridinylboronic acid complexes of diols were studied. (1) 3-pyridinylboronic acid is most useful among its regioisomers due to its availability and good water solubility; and (2) 3-pyridinylboronic acid binds to diols at physiological pH with the highest binding affinity for a monoarylboronic acid by NMR titrations and later by visible colorimetric competitive assays (Figure 2).

The surface enhanced Raman detection of glucose, was evaluated using a portable Raman Imaging Microscopy (PRIM). The PRIM probe head was custom-built by SpectraCode, Inc. It integrates in a SOX objective lens a center optic fiber for bringing in excitation light from a He-Ne laser (*35* mW, 632.8 urn, Melles Griot) and encircling thirty six optic fibers for high throughput collection of the scattering light from the sample to a thermoelectrically cooled CCD spectrometer ( InSpectrum®, Acton Research). The instrument control and data acquisition are achieved by SpectraSense software with a PC. The PRIM also includes a built-in video camera for viewing the sample on the PC screen by using a video frame grabbing PC-card. The PRIM probe head is clamped on an x-y-z adjustable stage for alignment to stationary samples. It can also be detached and hand held to the sample at a working distance of about one inch at any angle to obtain a Raman spectrum. The instrument is interfaced through a USB connection with a PC for instrument control, data acquisition and processing.

The instrument is incorporates spectral and video image collection optics, both coupled to the sample through the same objective lens. Thus observation of the "tattoo sensors" and acquisition of spectra can be performed simultaneously. The PRIM can be brought to a sample rather than requiring that the sample be set on a translation stage for alignment to a fixed microscope objective. The built-in video camera makes it possible to visually align the objective and thus ensure that spectra are collected from a precisely identified region in a sample.

An examination was made of the surface enhanced Raman spectra of these complexes using Raman sensing and imaging enhancement agents and the above referenced instrument along with the aid of standard ab initio calculations employ a dual-Xeon workstation and Gaussian 98 software. The surface enhancer is 60 nm gold nanoparticle aqueous solution using citrate as the stabilization agent. The results show very high sensitivity in the SERS detection of the pyridinylboronic acid in aqueous solution below 1 .0 ~iM. In addition, when glucose was added, significant spectroscopic changes were observed (Figure 3). From the MO theory calculation, the Raman active modes related to C-B bonds are thus assigned (Table 1). The peaks at 1341, *1558*, and *1586* cm⁻¹ are most visible.

For eventual *in vivo* applications as "tattoo dyes" of the optical sensors, it is necessary to encapsulate the sensors in a biocompatible polymer matrix. The second major technical achievement in this project is the development of narrowly dispersed core-shell or hollow spherical nanostructured polymer particles as the delivery devices for the sensors.

In is desirable to have a functional polymer interface within a biocompatible materials shell. The biocompatibility is achieved by using polymers that are approved by FDA for implants. The functional core is designed to interact with the "payloads" and the releasing of the payloads can be controlled chemically.

Boronic acids are known to complex with diols and 3-pyridinylboronic acid is an effective ionization agent for ESI-MS analysis of sugars (Scheme 1). Boronic acids form covalent complexes with sugars in water.

The most distinctive difference between glucose and fructose in the in situ derivatization ESI-MS experiments is the formation of intense signals of 1:2 gluose:1 complex ions 2, (355, m/z, positive mode Fig. 1a) and 3 (353, m/z, negative mode, Fig. 2a) for glucose. The 1:1 glucose:1 complex ions 4, (268, m/z, positive mode, Fig. 1a) and 5 (266, m/z, negative mode, Fig. 2a) were also present in the spectra. In contrast, only the 1:1 fructose:1 complex ions 6 and 7 (Scheme 3) were observed for fructose under the same ESI-MS conditions (Fig. 1b). In principle, glucose and fructose can thus be quantified simultaneously by a simple ESI-MS measurement. This capability is particularly important for blood glucose analysis in diabetic patients where fructose is often the main interference.

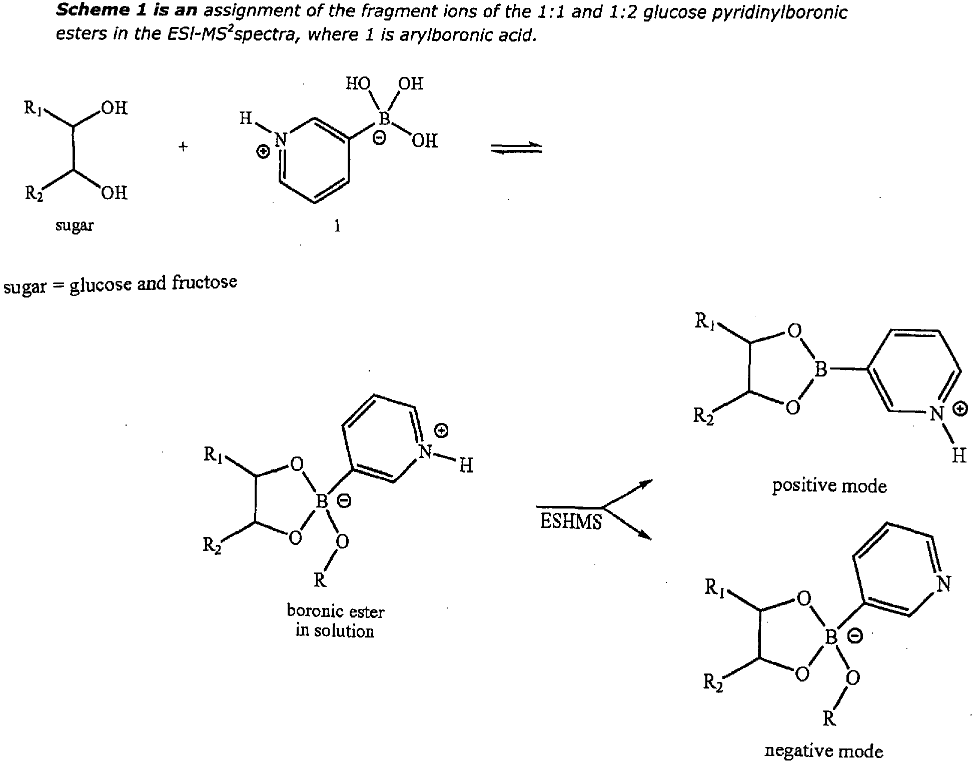

Glucose and fructose are isomeric monosaccharides that are important in managing diabetes as well as food and biotech processing and quality controlling. The exceptionally high sensitivity of mass spectrometry (MS) should allow blood glucose to be analyzed indirectly from body fluids such as tear or urine, if the isomers can be differentiated, for example, by simple electrospray ionization mass spectrometry (ESI-MS). With the same mass and similar functional groups, isomeric sugars are particularly challenge to be directly differentiated with mass spectrometry. Analysis is also difficult because there is little absorption or emission in the optical spectrum and because there are many stereoisomers.

This invention is not to be duly limited to the illustrative embodiments set forth herein.
1. A method comprising:
   analyzing a target analyte using laser excitation spectroscopy, colormetric analysis, or
   mass spectroscopy, wherein the analysis is enhanced using pyidinylboronic acid.
2. The method of item 1, wherein the pyridinylboronic acid is a Raman enhancement agent for detecting a polyhydroxyalcohol
3. The method of item 1, wherein the pyridinylboronic acid is an enhancement agent for detecting a polyhydroxyalcohol via mass spectroscopy.
3. The method of item 1, wherein the pridinylboronic acid is 3-pyridinyldihydroxy boronic acid
4. The method of item 1, wherein the pyridinylboronic acid is used as a Raman enhancement agent for Raman spectroscopy, hyper Raman spectroscopy, coherent anti-Stokes Raman spectroscopy or surface-enhanced Raman spectroscopy.
5. The method of item 2, wherein the polyhydroxyalcohol is glucose.
6. The method of item 4, wherein when the pyridinylboronic acid is used in surface-enhanced Raman spectroscopy, the nitrogen heteroatom of the pyridinylboronic acid is chemically attached to a noble-metal substrate.
7. The method of item 6, wherein the noble-metal substrate is gold.
8. The method of item 1 wherein the pyridinylboronic acid is used in mass spectrometry, electrospray ionization mass spectrometry, or combinations thereof.

## Claims

1. A method comprising detecting polyhydroxyalcohols in aqueous solutions using surface enhanced Raman spectroscopy, wherein the method is **characterized in that** pyridinylboronic acid is used as a Raman enhancement agent and the nitrogen heteroatom of the pyridinylboronic acid is chemically attached to a noble-metal substrate.

2. A method according to claim 1, wherein the noble-metal is gold.

3. A method according to any of claims 1 or 2, wherein the polyhydroxyalcohol is glucose.

4. A method according to any of claims 1 to 3, wherein the pyridinylboronic acid is encapsulated into a polymer shell.

5. A method according to claim 4, wherein the nanostructured polymer particles are used as delivery devices for sensors.

## Patentansprüche

1. Verfahren, das das Nachweisen von Polyhydroxyalkoholen in wässrigen Lösungen mit Hilfe von oberflächenverstärkter Raman-Spektroskopie umfasst, wobei das Verfahren **dadurch gekennzeichnet ist, dass** Pyridinylboronsäure als Raman-Verstärkungsmittel verwendet wird und das Stickstoff-Heteroatom der Pyridinylboronsäure chemisch an ein Edelmetallsubstrat gebunden ist.

2. Verfahren gemäß Anspruch 1, wobei es sich bei dem Edelmetall um Gold handelt.

3. Verfahren gemäß einem der Ansprüche 1 oder 2, wobei es sich bei dem Polyhydroxyalkohol um Glucose handelt.

4. Verfahren gemäß einem der Ansprüche 1 bis 3, wobei die Pyridinylboronsäure in eine Polymerhülle eingekapselt ist.

5. Verfahren gemäß Anspruch 4, wobei die nanostrukturierten Polymerteilchen als Abgabevorrichtungen für Sensoren verwendet werden.

## Revendications

1. Procédé comprenant la détection d'alcools polyhydroxylés dans des solutions aqueuses en utilisant la spectroscopie de Raman renforcée en surface, dans lequel le procédé est **caractérisé en ce que** l'on utilise de l'acide pyridinylboronique comme agent de renforcement Raman et l'hétéroatome d'azote de l'acide pyridinylboronique est chimiquement fixé à un substrat de métal noble.

2. Procédé selon la revendication 1, dans lequel le métal noble est l'or.

3. Procédé selon l'une quelconque des revendications 1 ou 2, dans lequel l'alcool polyhydroxylé est le glucose.

4. Procédé selon l'une quelconque des revendications 1 à 3, dans lequel l'acide pyridinylboronique est encapsulé dans une coque de polymère.

5. Procédé selon la revendication 4, dans lequel les particules de polymère à nanostructure sont utilisées comme dispositif de fourniture pour des capteurs.
